(19) Europäisches Patentamt
European Patent Office
Office européen des brevets

(11) Publication number: **0 456 287 A1**

(12) **EUROPEAN PATENT APPLICATION**

(21) Application number: **91200666.5**

(22) Date of filing: **25.03.91**

(51) Int. Cl.5: **C07C 403/22**, C07C 317/22, C07F 7/18, C07C 47/277

(30) Priority: **10.04.90 EP 90200863**

(43) Date of publication of application:
**13.11.91 Bulletin 91/46**

(84) Designated Contracting States:
**AT BE CH DE DK ES FR GB GR IT LI LU NL SE**

(71) Applicant: **DUPHAR INTERNATIONAL RESEARCH B.V**
**C.J. van Houtenlaan 36**
**NL-1380 AC Weesp(NL)**

(72) Inventor: **Lagerweij, Gerrit J., c/o OCTROOIBUREAU ZOAN B.V.**
**P.O. Box 140**
**NL-1380 AC Weesp(NL)**
Inventor: **Bakker, Cees, c/o OCTROOIBUREAU ZOAN B.V.**
**P.O. Box 140**
**NL-1380 AC Weesp(NL)**

(74) Representative: **Swaters, Pieter D. et al**
**OCTROOIBUREAU ZOAN B.V. P.O. Box 140**
**NL-1380 AC Weesp(NL)**

(54) **New sulfonyl compound and use of said compound in the process of producing vitamin A or its esters.**

(57) The invention relates to a new sulfonyl compound having the general formula

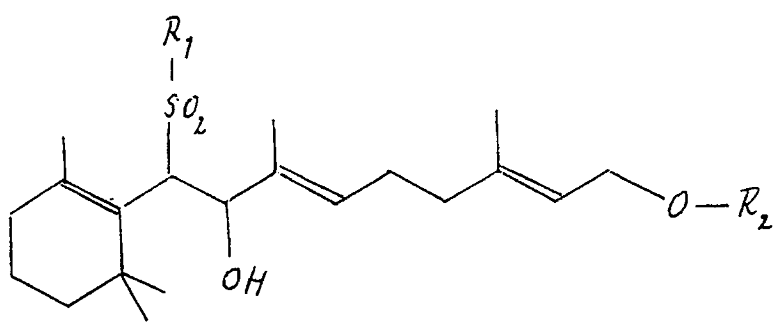

(I)

wherein
- $R_1$ is an unsubstituted or substituted aryl group; and
- $R_2$ is a $C_1$-$C_{20}$ alkyl group, a $C_2$-$C_{20}$ alkenyl group or a $C_2$-$C_{20}$ alkynyl group, which groups may be substituted with one or more substituents selected from the group consisting of $C_1$-$C_4$ alkoxy, halogen, unsubstituted aryl and substituted aryl; or wherein $R_2$ is a tri(hydrocarbyl)silyl group, an optionally lower-alkyl- substituted dihydro- or tetrahydropyran-2-yl group, an optionally lower- alkyl-substitute dihydro- or tetrahydro-fur-2-yl group, or a lower alkoxyalkylgroup.

This sulfonyl compound can be used as an intermediate in the process of producing vitamin A or its esters.

EP 0 456 287 A1

The invention relates to a new sulfonyl compound, to a method of preparing said compound and to the use of this sulfonyl compound as an intermediate in the process of producing vitamin A or its esters.

Vitamin A is essential for the normal growth of mammals and therefore can be used for treating a variety of disorders which are connected with a vitamin A deficiency or for preventing such disorders. Consequently, vitamin A and its esters, e.g., the acetate and the palmitate thereof, are used in great quantities as medicines, feed additives and food additives. It will hence be obvious that a good and convenient method of producing vitamin A is of great importance.

Rather recently Otera et al. have disclosed a new process for producing vitamin A or its esters and a useful intermediate for this process: European pat. appln. no. 0187259, J. Org. Chem. 51, 1986, 3830-3833, 3834-3838. The process described by Otera and co-workers is characterized in that a sulfonyl compound of the general formula

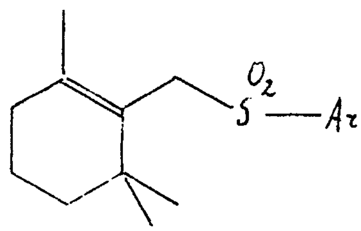

wherein Ar is an aryl group, is reacted with an aldehyde of the general formula

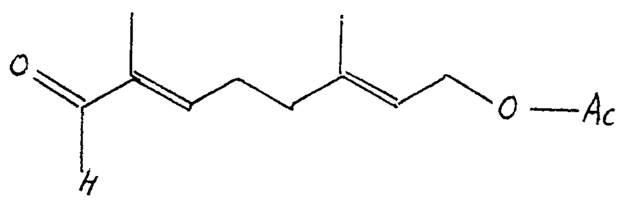

wherein Ac is a lower alkanoyl group, e.g., an acetyl group, after which the compound thus obtained, having the general formula

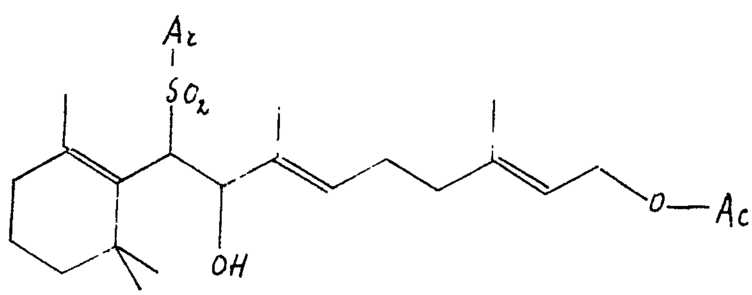

(IX)

is converted into a compound of the general formula

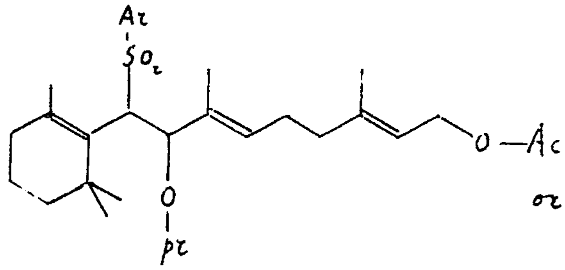

or

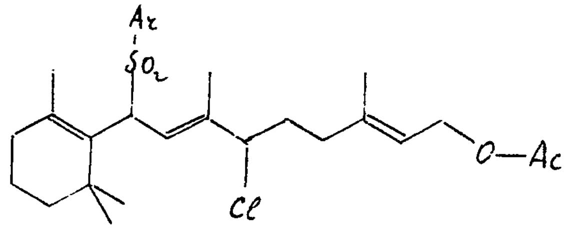

wherein pr is an acetal-type protective group, which compound is subjected to a double elimination reaction under the influence of a base, producing the all-trans structure of vitamin A in a high stereochemical purity.

Such a high stereochemical purity was not expected in view of an article of Mandai et al. (J. Am. Chem. Soc. 106, 1984, 3670-3672). In this article the double elimination of

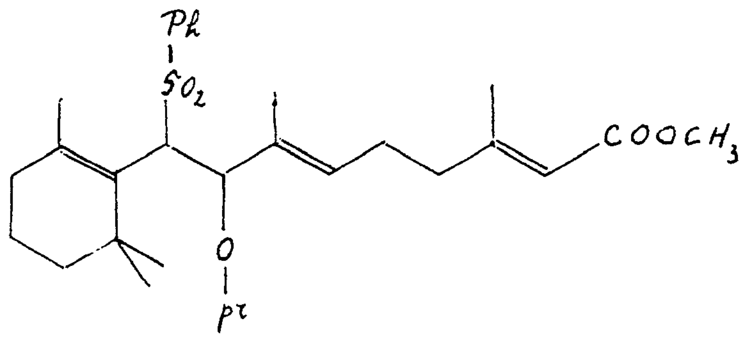

under the influence of a base is described, yielding the 13-cis and 13-trans isomers in a 1:1 ratio. So apparently the use of a terminal $CH_2$-O-Ac group is highly critical and essential for the stereospecific elimination reaction.

However, the formation of the key intermediate IX from the above sulfonyl compound and the above aldehyde is unsatisfactory with respect to the yield and the reaction conditions. The starting sulfonyl compound is used in excess with respect to the starting aldehyde; this excess of sulfonyl compound should be recovered in an after-treatment as completely as possible, because it is an expensive material. Therefore the yield is calculated not only on the quantity of aldehyde used, but also on the quantity of converted sulfonyl compound. when the reaction is carried out in the presence of a Grignard reagent, a yield of approximately 90% calculated on starting aldehyde can be obtained. It has appeared, that also the yield calculated on converted sulfonyl compound is approximately 90%. This means that during said known process both approximately 10% of starting aldehyde and approximately 10% of starting sulfonyl compound, both expensive materials, have been lost. During this reaction the reaction mixture should be cooled down to -40° C to -30° C. When the reaction is performed under the influence of n-butyllithium instead of a Grignard reagent, a few percents higher yield of the desired product of formula IX can be reached. Then the reaction should be carried out at -78° C to -50° C.

It will be evident that the above temperatures necessary to perform the desired reaction are difficult to realize in large-scale production. In addition, the loss of expensive materials, viz., approx. 10% of starting aldehyde and sulfonyl compound,in a rather early stage of the total vitamin A synthesis, is highly detrimental and increases the cost price of the vitamin.

It is the object of the present invention to provide a new method of producing vitamin A which does not present the above disadvantages.

This object can be achieved by using as a key intermediate in the vitamin A process a new sulfonyl compound which according to the present invention is presented by the general formula

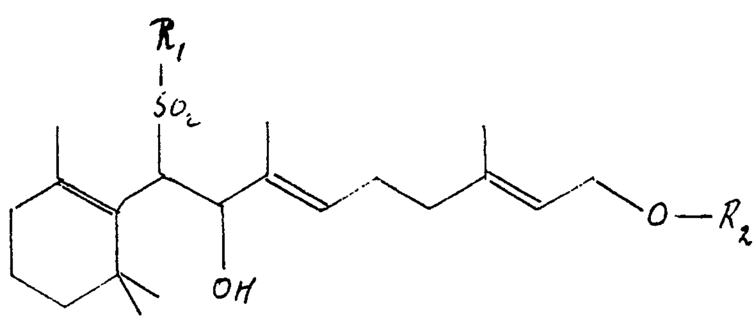

(I)

wherein $R_1$ is an unsubstituted or substituted aryl group;

and

$R_2$ is a $C_1$-$C_{20}$ alkyl group, a $C_2$-$C_{20}$ alkenyl group or a $C_2$-$c_{20}$ alkynyl groups, which groups may be

substituted with one or more substituents selected from the group consisting of $C_1$-$C_4$ alkoxy, halogen, unsubstituted aryl and substitued aryl; or wherein $R_2$ is a tri(hydrocarbyl)silyl group, an optionally lower-alkyl-substituted dihydro- or tetrahydropyran-2-yl group, an optionally lower-alkyl- substituted dihydro- or tetrahydro-fur-2-yl group, or a lower alkoxyalkyl group.

The above aryl group is preferably a phenyl group, but also other aryl groups are included, for example, pyridyl or thienyl. Said aryl group may be unsubstituted or substituted. Suitable substituents include lower alkyl, lower alkoxy and halogen, wherein the term "lower" alkyl means an alkyl group having 1-6 carbon atoms. Preferably three substituents at most are present on said aryl group. The above alkyl, alkenyl or alkynyl groups may be primary, secondary or tertiary alkyl, alkenyl or alkynyl groups. The term hydrocarbyl includes $C_1$-$C_8$ alkyl, $C_2$-$C_8$ alkenyl, $C_2$-$C_8$ alkynyl, phenyl and substituted phenyl.

It is indeed a surprise that the use of the above-defined group $R_2$ makes said key intermediate of formula I better accessible, starting with the basic sulfonyl compound and an aldehyde that is terminally provided with a corresponding $OR_2$-group. As a matter of fact, it has been found that said key intermediate of formula I can be prepared in an at least substantially quantitative yield, based both on starting aldehyde and on converted sulfonyl compound, under reaction conditions which are considerably less cumbersome than described by Otera et al. Even at a reaction temperature of approx. 20° C the reaction between the starting sulfonyl compound and the above aldehyde in the presence of a Grignard reagent as a base proceeds smoothly and yields the compound of formula I quantitatively.

The key intermediate of the present invention is preferably characterized by the general formula

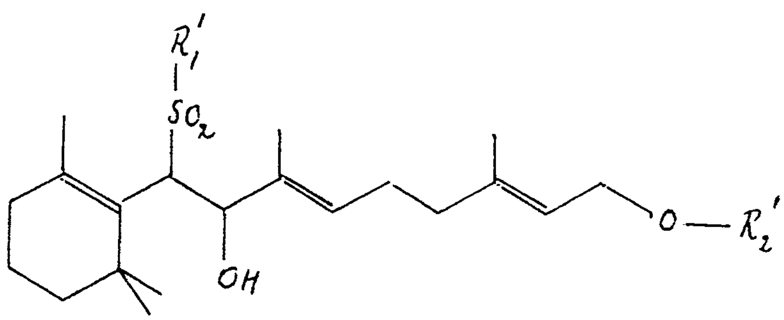

(II)

wherein

- $R_1$' is an unsubstituted phenyl group or a phenyl group substituted with 1 - 3 substituents selected from the group consisting of lower alkyl, lower alkoxy and halogen; and
- $R_2$' is a tri(hydrocarbyl)silyl group, wherein hydrocarbyl is selected from the group consisting of $C_1$-$C_8$ alkyl and phenyl; or wherein
- $R_2$' is a methyl group, a tert. ($C_4$-$C_{12}$)alkyl group, a tetrahydropyran-2-yl group, a tetrahydrofur-2-yl group, or an ethoxyethyl group.

As stated above, the key intermediate of general formula I can easily be obtained from the starting sulfonyl compound and the aldehyde in question. Consequently, the present invention also relates to a method of preparing said intermediate by reacting a sulfonyl compound of the general formula

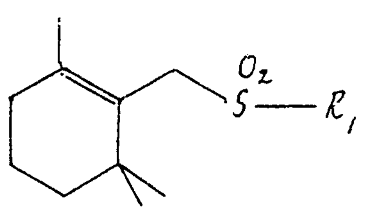

(III)

wherein $R_1$ has the above meaning,
with an aldehyde of the general formula

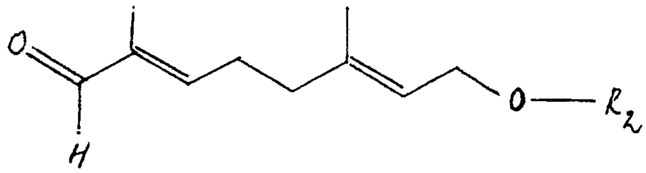

(IV)

wherein $R_2$ also has the above meaning, with the aid of a base.

Suitable bases are, for example, magnesium compounds, e.g., having the general formula $R_5MgY$, wherein $R_5$ is an alkyl group, an aryl group, an alkenyl group or a dialkylamino group, and Y is Cl or Br. Other bases that may be used are lithium compounds of the general formula $R_5Li$ like methyllithium and n-butyllithium, as well as lithiumdiisopropylamide. In case $R_5$ is or comprises an alkyl group, this alkyl group preferably comprises 1 to 6 carbon atoms; if $R_5$ is an aryl group, said aryl group is preferably phenyl. Preferably both the starting compound III and the base are in excess with respect to the starting aldehyde IV to reach the best results. The starting aldehyde having the general formula IV can easily be obtained from the corresponding alcohol (compound formula IV, $R_2$ = H), e.g., by a reaction with a suitable silyl halogenide, a suitable alkylating agent, or a suitable, whether or not cyclic acetalization agent.

Alternatively, the aldehyde of the general formula IV can conveniently be prepared by a selenium dioxide oxidation from the corresponding geranyl ether having the general formula

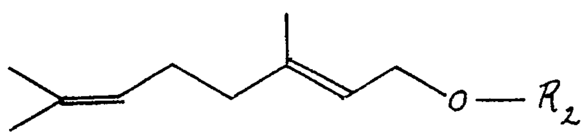

wherein $R_2$ has the above meaning.

In case $R_2$ is a (substituted) alkyl - alkenyl- or alkynyl group or represents a tri(hydrocarbyl)silyl group, the above aldehyde is new. Therefore, the invention also relates to an aldehyde compound, suitable for the above-described preparation method, having the general formula

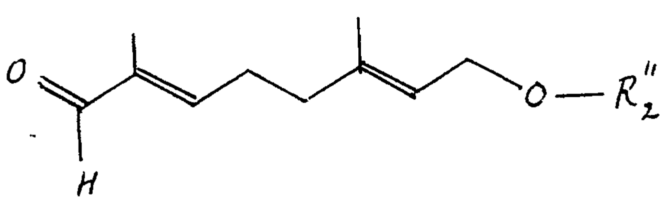

(XI)

wherein $R_2''$ is a $C_1$-$C_{20}$ alkyl group, a $C_2$-$C_{20}$ alkenyl group or a $C_2$-$C_{20}$ alkynl groups, which groups may be substituted with one or more substituents selected from the group consisting of $C_1$-$C_4$ alkoxy, halogen, unsubstituted aryl and substituted aryl, preferably a methyl group or a tert. ($C_4$-$C_{12}$)alkyl group; or wherein $R_2''$ is a tri(hydrocarbyl)silyl group, wherein hydrocarbyl is selected from the group consisting of $C_1$ - $C_8$ alkyl and phenyl.

It has been found, that the key intermediate defined above, i.e., the sulfonyl compound of general formula I, is excellently suitable as a starting material for the preparation of vitamin A or an ester thereof. Four different reaction routes are suitable to reach the desired result, each of them being composed of a number of successive synthetic steps.

The free hydroxy group in the 8-position of the compound of formula I can first be protected by an acetal-type protective group generally known for protecting an hydroxy group. Then a compound is formed having the general formula

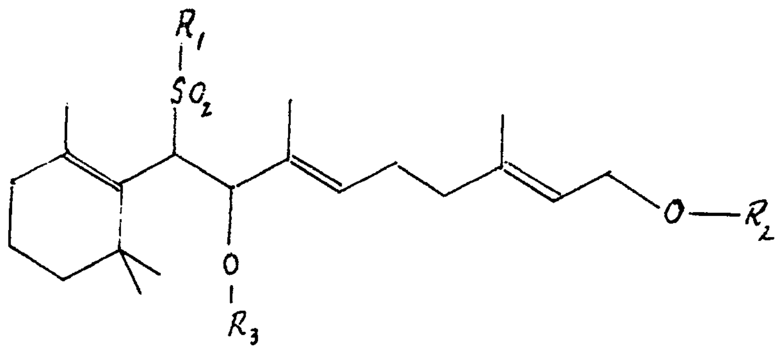

(V)

wherein

| | |
|---|---|
| $R_1$ and $R_2$ | have the above meanings, and |
| $R_3$ | is an optionally lower-alkyl-substituted dihydro- or tetrahydro-pyran-2-yl group, an optionally lower-alkyl-substituted dihydro-or tetrahydro-fur-2-yl group, or a lower alkoxyalkyl group; |

Alternatively, said compound of formula I may be reacted with a suitable halogenating agent like a thionylhalogenide, a phosphoroxyhalogenide or a phosphorhalogenide, preferably in the presence of a base, to form a compound of the general formula

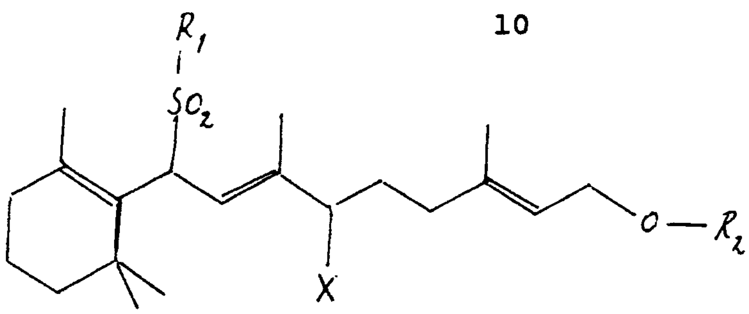

(VIII)

wherein

| | |
|---|---|
| $R_1$ and $R_2$ | have the above meanings, and |
| X | represents a halogen atom. |

The above compounds of formulas V and VIII are new compounds.

In a subsequent reaction step the group $R_2$ can be removed, both from the compound of formula V and from the compound of formula VIII, by converting the group $OR_2$ in said compound into a group $OR_4$, wherein $R_4$ is a hydrogen atom or a lower alkanoyl group. For example, conversion into hydrogen can be performed with a suitable acid, and conversion into an acetyl group can conveniently be achieved with a suitable acetylating agent, e.g., acetic anhydride, in the presence of a Lewis acid like $FeCl_3$, preferably in a polar inert organic solvent like acetonitrile. Thereupon the compound thus obtained can finally be converted into vitamin A or an ester thereof by a double elimination reaction as described in the publications of Otera at al., mentioned hereinbefore.

Alternatively, in a subsequent reaction the compound of formula V or VIII has proved to be prone to undergo a double elimination reaction, the compound of formula VIII in two steps, resulting in a vitamin A derivative of the general formula VII:

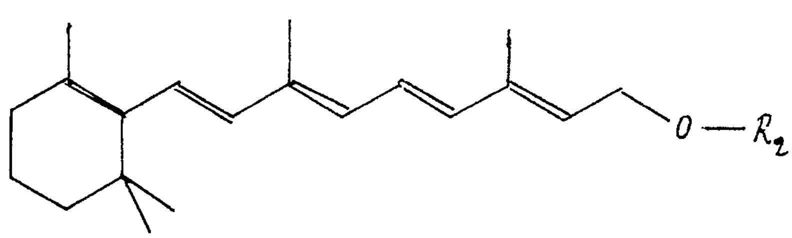

(VII)

In this compound of formula VII $R_2$ can be removed by converting the group $OR_2$ into a group $OR_4$ by using a suitable deprotecting agent.

If desired, said double elimination reaction can also be performed in two separate steps with an intermediate deprotection of $OR_2$ (conversion of $OR_2$ into $OR_4$). The first elimination, which should be carried out under the influence of a suitable base, e.g. an aza-compound such as 1,5 - diazabicyclo[5,4,0]-undecene-5, resulting in an intermediate of the general formula

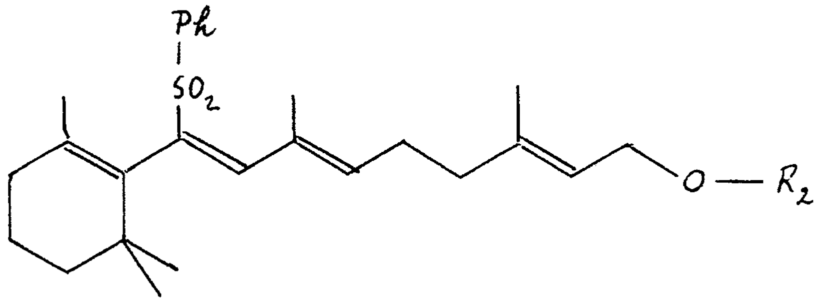

(X)

This intermediate, after conversion of $OR_2$ to $OR_4$, can be converted by a further elimination reaction with the aid of a base, as defined below for the double elimination reaction, yielding vitamin A or an ester thereof.

As stated before, the double elimination reaction is a highly critical step in the vitamin A synthesis, because by this reaction the all-trans structure of vitamin A should be produced. As said before, the required stereospecificity of the double elimination reaction depends on the terminal group: the starting acetate compound yields the desired all-trans configuration, whereas the starting acid ester compound, on the contrary, yields in the same elimination reaction a 1:1 mixture of 13-cis and 13-trans isomers. In view of this it is quite a surprise, that the double elimination of both the compound of formula V and the compound of formula VIII, both provided with a terminal $OR_2$group, proceeds stereospecifically to the all-trans configuration. Said double elimination reaction can be carried out under the influence of a suitable base, e.g., potassium methoxide or potassium hydroxide, in an inert organic solvent like a hydrocarbon, e.g., hexane or toluene.

The invention finally relates to a method of preparing vitamin A or an ester thereof, by first preparing the sulfonyl compound of general formula I from the starting compound of formula III and the aldehyde of formula IV, followed by a conversion of said formula I compound into the desired vitamin via any of the reaction routes as discussed above.

The invention will now be described in greater detail with reference to the following specific examples.

EXAMPLE I

Preparation of 1-(dimethyl-t.alkyl)silyloxy-8-hydroxy-3,7-dimethyl-9-(2,6,6-trimethyl-1-cyclohexen-1-yl)-9 phenylsulfonyl-2(E),6(E)-nonadiene

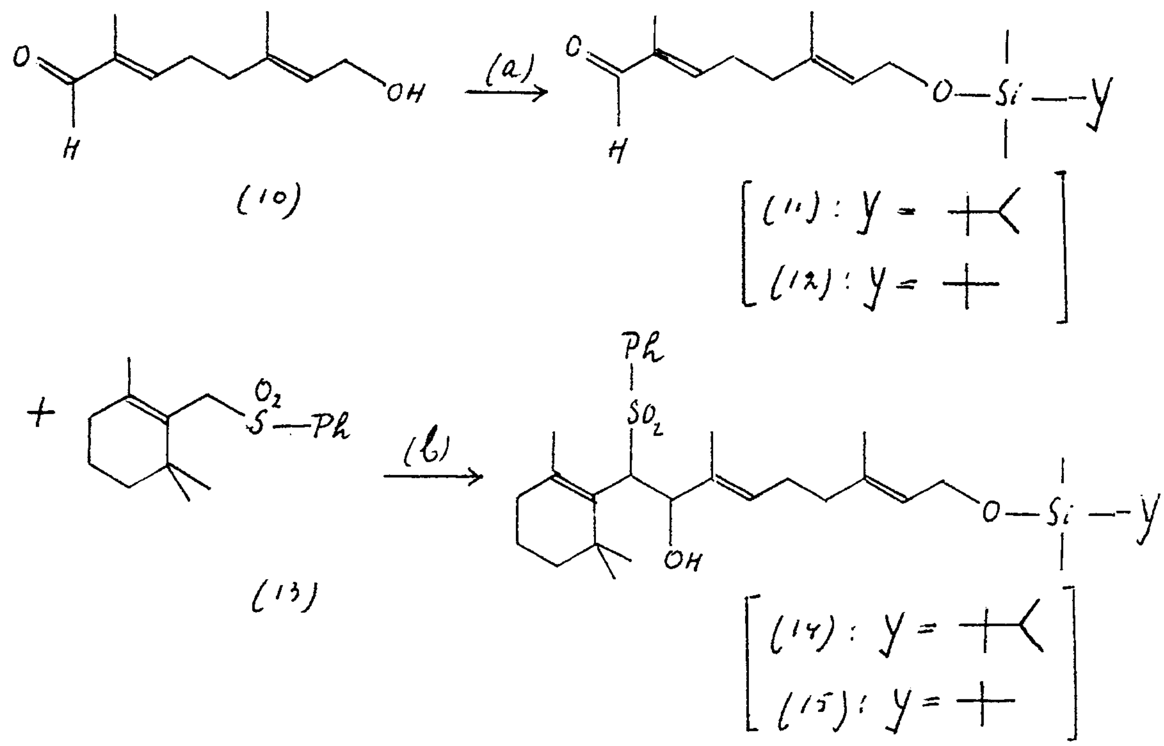


(10)

(11)

(12)

(13)

(14)

(15)

(a) The aldehyde (10) is dissolved in a quantity of 17.4 g (103 mmol) into 100 ml of methylene chloride; to this solution are added successively 15.6 ml (113 mmol) of triethylamine and 0.5 g (4.1 mmol) of dimethylaminopyridine. To the solution thus obtained is added dropwise a solution of 20.3 ml (103 mmol) dimethyl-1,1,3-trimethylpropyl silylchloride in 30 ml of methylene chloride in 5 min at ambient temperature. The reaction mixture is stirred overnight, after which 100 ml of water is added. Separation, washing of the organic layer with aqueous ammoniumchloride, drying and evaporation yields the desired compound (11) as an oily substance in a yield of 29.9 g. Purification by flash chromatography (hexane/ethyl acetate). Identification by NMR:
$^1$H-NMR ($CDCl_3$): 0.10(s.6H); 0.85(s.6H); 0.88(d.6H); 1.62(m.1H); 1.65(s.3H); 1.75(s.3H); 2.19(t.2H); 2.49-(m.2H); 4.18(d.2H); 5.35(t.1H); 6.47(t.1H); 9.38(s.1H).
In a corresponding manner compound (12) is prepared. Identification by NMR:
$^1$H-HMR($CDCl_3$): 0.07(s.6H); 0.91(s.9H); 1.64-1.66(2s.3H); 1.75-1.77(2s.3H); 2.18(m.2H); 2.49-2.70(2m.2H); 4.20(m.2H); 5.35(m.1H); 6.48(m.1H); 9.38-10.13(2s.1H).

(b) To a solution of 2.84 g (10.21 mmol) of compound (13) in 7 ml of tetrahydrofuran is added dropwise 4.0 ml (9.48 mmol) of isopropylmagnesiumchloride. The mixture is heated on 35°C during 3 h. After cooling down to approx. 20°C, to this mixture is added dropwise a solution of 2.28 g (7.35 mmol) of the above obtained compound (11) in 10 ml of tetrahydrofuran. The reaction mixture is stirred for 2 h at approx. 20°C and then successively diluted with 30 ml of diethyl-ether and a cold solution of 0.7 ml concentrated sulphuric acid in 40 ml water. After stirring for 5 min the layers are separated and the acid water layer is extracted with diethylether.
Thereupon the combined organic layers are washed successively with a bicarbonate solution and with water (twice), dried and evaporated. The final product (5.15 g) contains 82% of the desired title compound (14) and 16% of compound (13) according to $^1$H-NMR analysis. The yield of the desired compound (14) is 100% based on aldehyde (11) and 100% based on converted sulfonyl compound (13).
Compound (14) is isolated as a pure substance by flash chromatography: $^1$H-HMR ($CDCl_3$): 0.10(s.6H); 0.69(s.3H); 0.85(s.6H); 0.89(d.6H); 0.89(s.3H); 1.53(s.3H); 1.60(s.3H); 2.01(s.3H); 1.3-2.3(11H); 3.77 (d.1H); 4.03 (d.1H); 4.16(d.2H); 5.04(dd.1H); 5.28 (t.1H); 5.33 (t.1H); 7.53(t.2H); 7.60(t.1H); 8.05(d.2H).
In a corresponding manner compound (15) is prepared, equally in a yield of 100% based on aldehyde (12). $^1$N-NMR($CDCl_3$):
0.07(s.6H); 0.69(s.3H); 0.91(2s.12H); 1.53(s.3H); 1.61(s.3H); 1.3-1.8(4H); 1.9-2.3(6H); 2.01(s.3H); 3.81-(d.1H); 4.02(d.1H); 4.18(d.2H); 5.05(dd.1H); 5.29(t.1H); 5.34 (t.1H); 7.51(t.2H); 7.58(t.1H); 8.04(d.2H).

EXAMPLE II

Preparation of the tetrahydropyran-2-yl ether and the tert. butylether

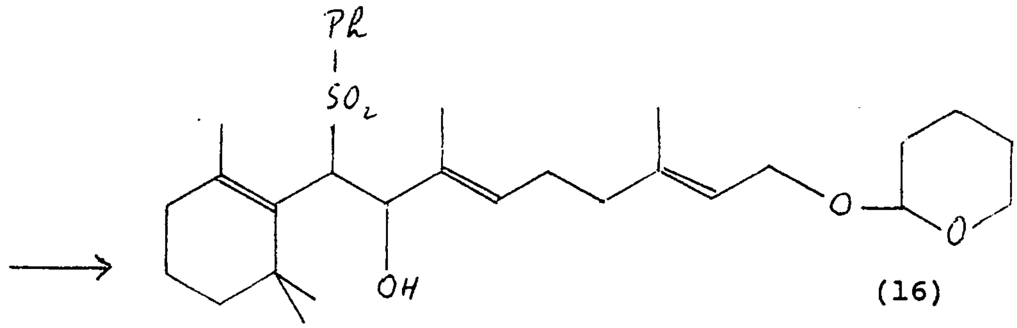

(16)

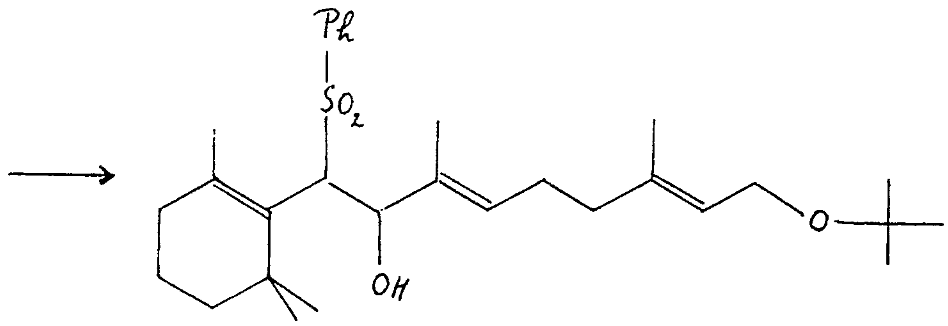

(17)

In a corresponding manner as described in Example I the above two ethers are prepared in yields of 99% and 100% respectively, calculated on the corresponding aldehydes. Identification by their NMR spectra:
compound no.(16): $^1$N-NMR($CDCl_3$): 0.69(s.3H); 0.89(s.3H); 1.53(s.3H); 1.66(s.3H); 2.01(s.3H); 1.3-2.3(16H); 3.53(m.1H); 3.79(t.1H); 3.88(m.1H); 4.00(m.1H); 4.02(d.1H); 4.24(m.1H); 4.63(t.1H); 5.05(dd.1H); 5.33(t.1H); 5.35(t.1H); 7.54(t.2H); 7.61(t.1H); 8.04(d.2H).
compound no. (17): $^1$H-NMR($CDCl_3$): 0.68(s.3H); 0.89(s.3H); 1.22(s.9H); 1.53(s.3H); 1.64(s.3H); 2.00(s.3H); 1.3-2.3(10H); 3.78(d.1H); 3.90(d.2H); 4.02(d.1H); 5.04(dd.1H); 5.30(t.1H); 5.33(t.1H); 7.54(t.2H); 7.60(t.1H); 8.04(d.2H).

The starting aldehyde for the above tert.butylether (17), having the formula

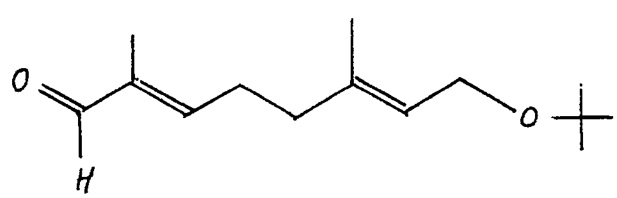

(18)

is prepared as follows.

A quantity of 351.2 mg geraniolaldehyde (10) and 487.6 mg t.-butyltrichloroacetimidate are dissolved in a mixture of 3ml hexane and 2ml dichloromethane. After cooling down to 0°C, one drop of boriumfluoride -etherate is added. The reaction mixture is stirred at 0°C for 1hr. Purification by flash chromatography yields the desired product (18) in a yield of 70.2 mg. Identification by NMR:
$^1$H- NMR ($CDCl_3$): 1.23(s.9H); 1.69(s.3H); 1.76(s.3H); 2.21(m.2H); 2.50(m.2H); 3.93(d.2H); 5.37(t.1H); 6.48-(t.1H); 9.40(s.1H).

EXAMPLE III

(a) Preparation of 1-(dimethyl-t.alkyl)-silyloxy-6-chloro-3,7-dimethyl-9-(2,6,6-trimethyl-l- cyclohexen-l-yl)-9-phenylsulfonyl-2,7-nonadiene.

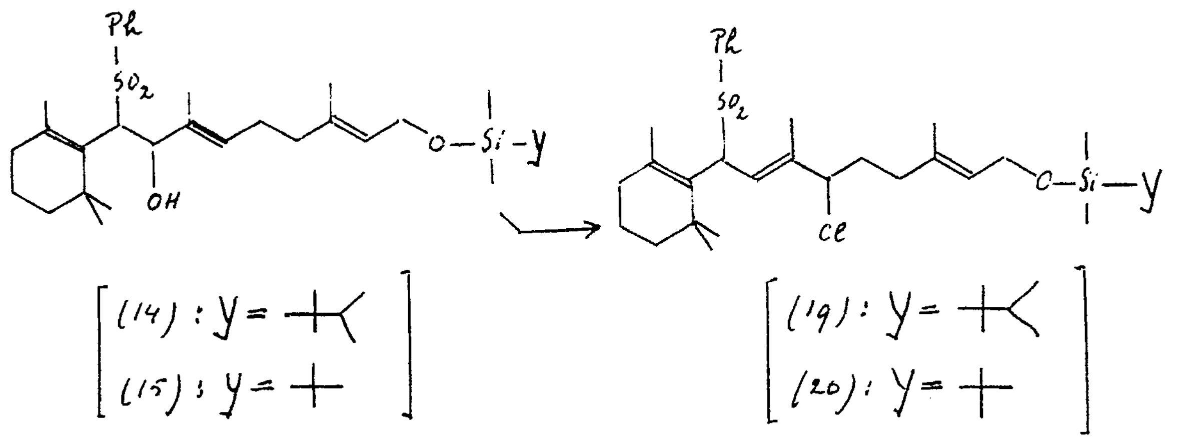

(14) (15) (19) (20)

The silylether prepared according to Example I (14) is converted as shown above by dissolving 8.98 g (15.26 mmol) into 45 ml of dry toluene. To this solution, cooled down to -10 °C, is added successively 2.70 ml (33.6 mmol) of pyridine and a solution of 1.23 ml (16.8 mmol) of thionylchloride in 10 ml of toluene. After stirring for 1 h at -5° to 0 °C the reaction is poured onto a mixture of 50 ml diethylether, 50 ml 5% sodium bicarbonate solution and 50 ml water. The layers are separated and washed. The combined organic layer is evaporated yielding 8.21 g of compound (19). Identification by means of NMR:

$^1$H-NMR($CDCl_3$): 0.09(s.6H); 0.77(s.3H); 0.84(s.6H); 0.87(d.6H); 0.93(d.3H); 1.20(s.3H). 1.58(s.3H); 2.09(s.3H); 1.3-2.3(11H); 4.13(t.2H); 4.33(t.1H); 4.48(d.1H); 5.24(t.1H); 5.94(d.1H); 7.54(t.2H); 7.63(t.1H); 7.89(d.2H).

In a corresponding manner compound (20) is prepared.

$^1$H-NMR($CDCl_3$): 0.07(s.6H); 0.78(s.3H); 0.9(s.9H); 0.93(s.3H); 1.2(s.3H); 1.58(s.3H); 2.09(s.3H); 1.3-2.3(10H); 4.1-4.2(2H); 4.34(t.1H); 4.48(d.1H); 5.25(t.1H); 5.94(d.1H); 7.52(t.2H) 7.61(t.1H); 7.88(d.2H).

(b)

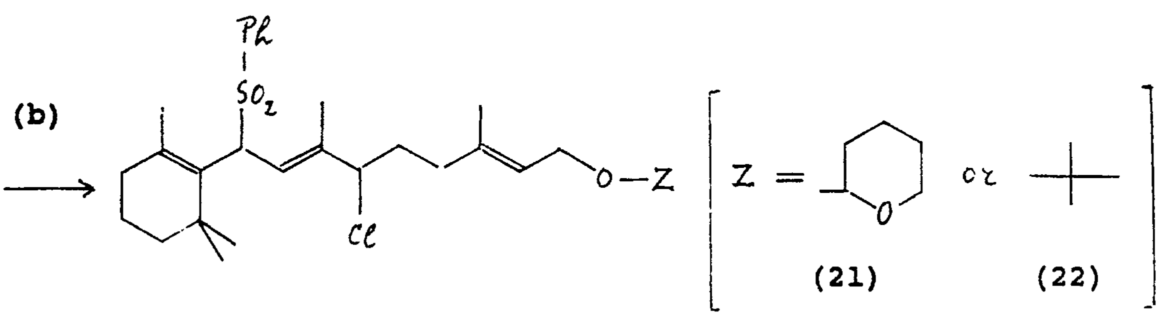

(21) (22)

In a corresponding manner as described above sub (a) the above 6-chloro compounds are prepared and identified by NMR.

compound no. (21): $^1$H-NMR($CDCl_3$): 0.77(s.3H); 0.93(d.3H); 1.20(s.3H); 1.64(s.3H); 2.10(s.3H); 1.3-2.3(16H); 3.51(m.1H); 3.87(m.1H); 3.97(m.1H); 4.23(m.1H); 4.35(t.1H); 4.48(d.1H); 4.60(t.1H); 5.30(t.1H); 5.94(d.1H); 7.54(t.2H); 7.63(t.1H); 7.90(d.2H).

compound no. (22): $^1$H-NMR($CDCl_3$): O.77(s.3H); 0.92(d.3H); 1.19(s.3H); 1.22(s.9H); 1.62(s.3H); 2.10(s.3H); 1.3-2.3(10H); 3.88(t.2H); 4.34(t.1H); 4.47(d.1H); 5.24(t.1H); 5.94(d.1H); 7.54(t.2H); 7.63(t.1H); 7.89(d.2H).

EXAMPLE IV

Preparation of 1-acetoxy-6-chloro-3,7-dimethyl-9-(2,6,6-trimethyl-1-cyclohexen-l-yl)-9-phenylsulfonyl-2,7-nonadiene (23).

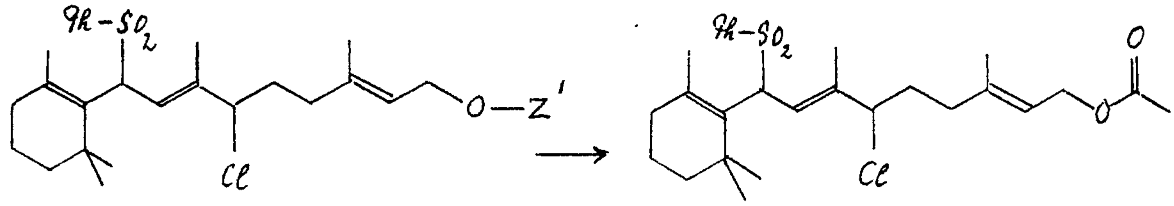

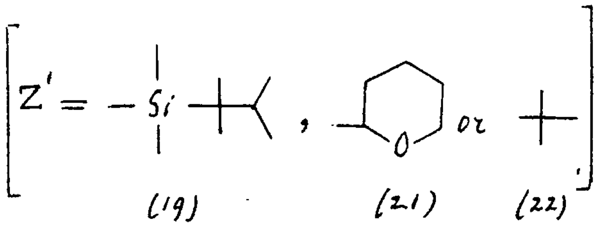

(19) (21) (22)

(23)

The compound prepared according to Example III (a) (19) is converted as indicated above by dissolving 1.0189 g (1.68 mmol) in 10 ml of acetonitrile. To this solution are successively added 0.79 ml (8.4 mmol) of acetic anhydride and 3.6 ml (0.166 mmol) of a $FeCl_3$-solution, obtained by dissolving 0.75 g of $FeCl_3$ in 100 ml of acetonitrile, After stirring at 50° C for 4 h, the reaction mixture is cooled down to room temperature and then treated with a saturated $Na_2HPO_4$ solution. Dilution with diethylether, separation and washing gives an organic phase which, after drying and evaporation, yields the desired compound (23) in a yield of 0.9600 g. Identification by NMR: $^1$H-NMR ($CDCl_3$): 0.78(s.3H); 0.94(d.3H); 1.20(s.3H); 1.67(s.3H); 2.06(s.3H); 2.10-(s.3H); 1.3-2.3(10H); 4.33(t.1H); 4.48(d.1H); 4.55(dd.2H); 5.28(t.1H); 5.94(d.1H); 7.55(t.2H); 7.63(t.1H); 7.89-(d.2H).

In a corresponding manner the compounds (21) and (22), prepared according to Example III (b), are converted into compound (23).

If desired, compound (23) can be converted into vitamin A acetate exactly as described in the publication of Otera et al., mentioned before.

EXAMPLE V

Preparation of vitamin A dimethyl-t.alkylsilyl ether.

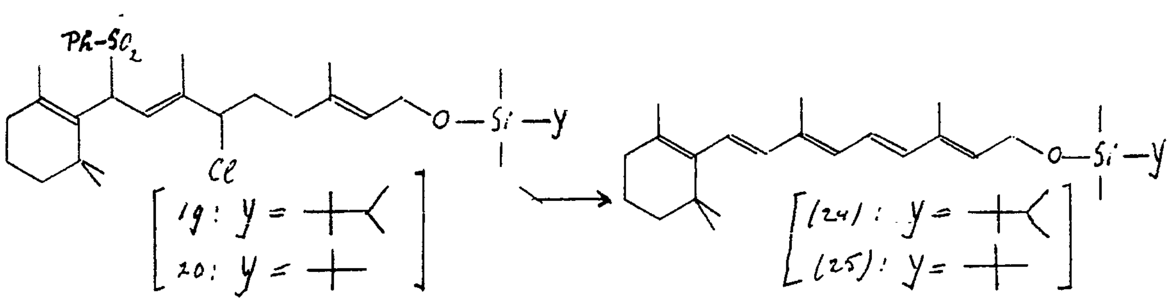

The compound prepared according to Example III (a) (19) is subjected to a double elimination reaction as shown above by dissolving 960.7 mg (1.58 mmol) of this compound into 5 ml of hexane. To this solution are successively added 443 mg (6.34 mmol) of potassium methoxide and 5 ml of hexane. The reaction mixture is refluxed for 2 h and then cooled down to room temperature. After addition of 20 ml of diethylether and 20 ml of water, the mixture is stirred for 5 min and then the layers are separated. The organic layer is washed successively with water, aqueous bicarbonate and water and then dried. After evaporation, the desired vitamin A ether (24) is obtained in a yield of 617.7 mg (98%); all-trans content 94,1% (NMR). $^1$N-NMR ($CDCl_3$): 0.11(s.6H); 0.85(s.6H); 0.89(d.6H); 1.02(s.6H); 1.71(s.3H); 1.81(s.3H); 1.94-(s.3H); 1.35-2.15(7H); 4.33(d.2H); 5.60(t.1H); 6.09(dd.1H); 6. 15(d.1H); 6.28(d.1H); 6.55(dd.1H).

In a corresponding manner compound (25) is prepared as a solid substance; $^1$H-NMR($CDCl_3$): 0.09-(s.6H); 0.91(s.9H); 1.02(s.6H); 1.46(m.2H); 1.61(m.2H); 1.71(s.3H); 1.81(s.3H); 1.95(s.3H); 2.01(t.2H); 4.34-

(d.2H); 5.61(t.1H); 6.09(2d.2H); 6.15(d.1H); 6.27(d.1H); 6.56(dd.1H).

EXAMPLE VI

Preparation of vitamin A tetrahydropyran-2-yl ether and of vitamin A tert. butyl ether.

(21) 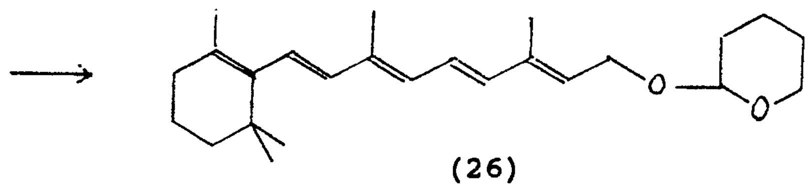

(26)

(22) 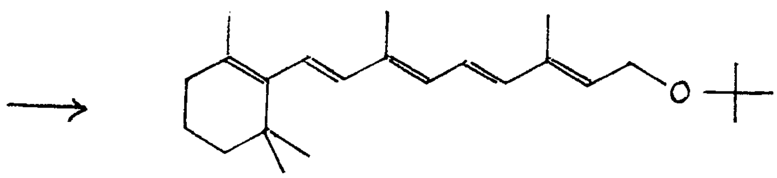

(27)

In a corresponding manner as described in Example V the 6-chloro compounds (21) and (22), prepared according to Example III (b), are converted in a double elimination reaction to the corresponding vitamin A ethers: (26) and (27), respectively.
Identification by NMR:
compound (26): $^1$H-NMR($CDCl_3$): 1.02(s.6H); 1.4-1.65(4H); 1.71(s.3H); 1.86(s.3H); 1.95(s.3H); 2.02(t.2H); 1.3-2.1(6H); 3.51(m.1H); 3.9O(m.1H); 4.19(m.1H); 4.38(m.1H); 4.65(t.1H); 5.66(t.1H); 6.10(d.1H); 6.12(d.1H); 6.16-(d.1H); 6.30(d.1H); 6.60(dd.1H);
compound (27): $^1$H-NMR($CDCl_3$): 1.02(s.6H); 1.24(s.9H); 1.46(m.2H); 1.62(m.2H); 1.71(s.3H); 1.84(s.3H); 1.95(s.3H); 2.02(t.2H); 4.08(d.2H); 5.62(t.1H); 6.09(d.1H); 6.10(d.1H); 6.15(d.1H); 6.28(d.1H); 6.56(dd.1H).
The yields are substantially quantitative. The all-trans content of compound (26) is 94%, that of compound (27) 97% (NMR).

EXAMPLE VII

Preparation of vitamin A and its acetate.

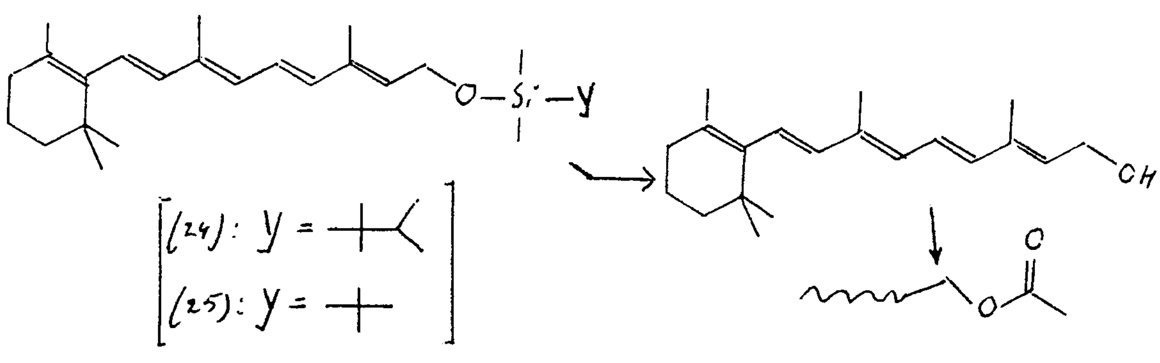

The vitamin A silyl ether prepared according to Example V (24) is converted as indicated above into vitamin A and its acetate by dissolving 1.0129 g (2.37 mmol) into 10 ml of methylene chloride. To this solution, cooled down to 0 °C, a solution of 0.92 g (3.5 mmol) of tetrabutylammonium fluoride in 10 ml of methylene chloride is added at once. After stirring for 4 h the reaction mixture is diluted with 20 ml of hexane and washed twice with 20 ml of a concentrated ammoniumchloride solution. Drying and evaporation yields vitamin A in a yield of 0.95 g. In a corresponding manner vitamin A silyl ether (25) is converted to

vitamin A.

Vitamin A is converted directly in its acetate by dissolving 0.95 g (2.4 mmol) of vitamin A in 15 ml of hexane. To this solution are added successively 1.70 ml (12.3 mmol) of triethylamine and a solution of 1.12 ml (11.9 mmol) of acetic anhydride in 5 ml of hexane dropwise. After stirring overnight at room temperature, the reaction mixture is diluted with 20 ml of hexane and then stirred with 20 ml saturated ammoniumchloride solution. After separation the organic layer is washed with a saturated ammoniumchloride solution, dried and evaporated. Vitamin A acetate is obtained as an oil in a yield of 0.9875 g. HPLC-analysis: overall yield 82%; all-trans content 90.9%.

$^1$H-NMR($CDCl_3$): 1.02(s.6H); 1.47(m.2H); 1.62(m.2H); 1.71(s.3H); 1.89(s.3H); 1.96(s.3H); 2.01(t.2H); 2.06-(s.3H); 4.73(d.2H); 5.61(t.1H); 6.09(d.1H); 6.11(d.1H); 6.18(d.1H); 6.28(d.1H); 6.64(dd.1H).

EXAMPLE VIII

Preparation of 1-hydroxy-3,7-dimethyl-9-(2,6,6-trimethyl-1-cyclohexen-1-yl)-9-phenylsulfonyl-2,6,8-nonatriene(29)

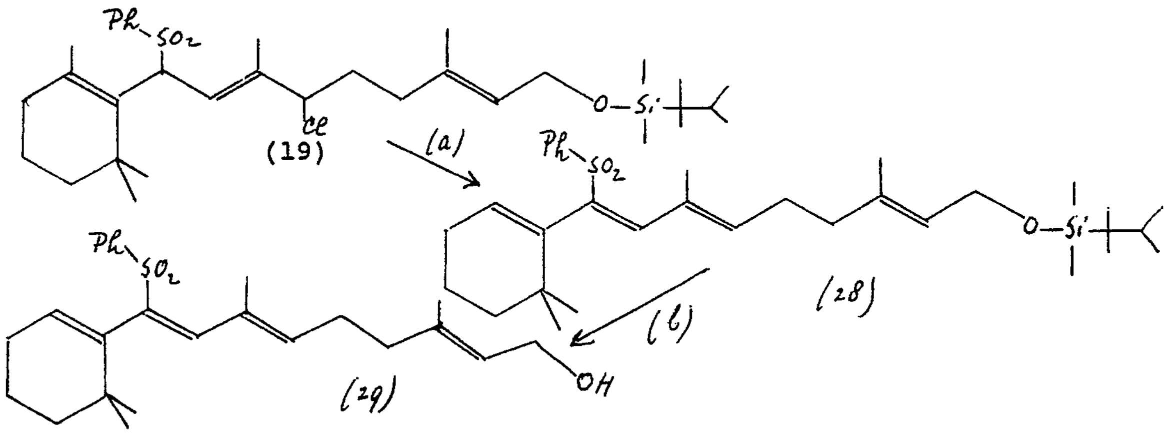


(a) Compound (19), prepared according to Example III(a), in a quantity of 5.55g (9.15mmol) is dissolved into 100 ml of dry diethylether. To the solution are added 2.73ml (18.3mmol) of 1.5 - diazabicyclo[5.4.0]-undecene-5 and the mixture is refluxed for 18.5 hr. The precipitate is sucked off and the filtrate is washed successively with 0.5% sulphuric acid (2x), aqueous sodium bicarbonate solution and water (2x). Drying and evaporation yields the desired compound (28) in a yield of 4.86g. The product is purified by flash chromatography (eluens:hexane/ethyl acetate = 9/1) and identified by its NMR spectrum. $^1$H-NMR-($CDCl_3$):
0.11(s.6H); 0.85(s.6H); 0.89(d.6H); 0.95(s.3H); 1.03(s.3H); 1.28(s.3H); 1.61(s.3H); 1.67(s.3H); 1.1-2.3(11H); 4.17(d.2H); 5.29(t.1H); 5.83(t.1H); 7.22(s.1H); 7.49(t.2H); 7.57(t.1H); 7.87(d.2H).

(b) The above-prepared compound (28) is deprotected by dissolving 2.50g (3.20mmol) in 40ml of tetrahydrofuran. After cooling down to approx. 0°C, to this solution is added a solution of 2.30g (8.8mmol) of tetrabutylammoniumfluoride in 15ml of tetrahydrofuran. After stirring at 0°C for 1hr and overnight at room temperature, the reaction mixture is diluted with 50ml of diethylether and subsequently washed twice with saturated aqueous $NH_4Cl$-solution. After drying and evaporation the desired product (29) is obtained in a yield of 2.31g. Flash chromatography (hexane/ethyl acetate = 1/1) yields the pure product, which is identified by NMR.

$^1$H-NMR($CDCl_3$): 0.95(s.3H); 1.01(s.3H); 1.28(s.3H); 1.65(s.3H); 1.67(s.3H); 0.7-2.35(10H); 2.55(s.1H); 4.13(d.2H); 5.39(t.1H); 5.82(t.1H); 7.21(s.1H); 7.49(t.2H); 7.56(t.1H); 7.86(d.2H).

Compound (29) is converted into vitamin A as described in the publication of Otera et al., mentioned before, with the aid of potassium methoxide.

EXAMPLE IX

Preparation of vitamin A dimethyl-l,1,3-trimethylpropylsilyl ether (24)

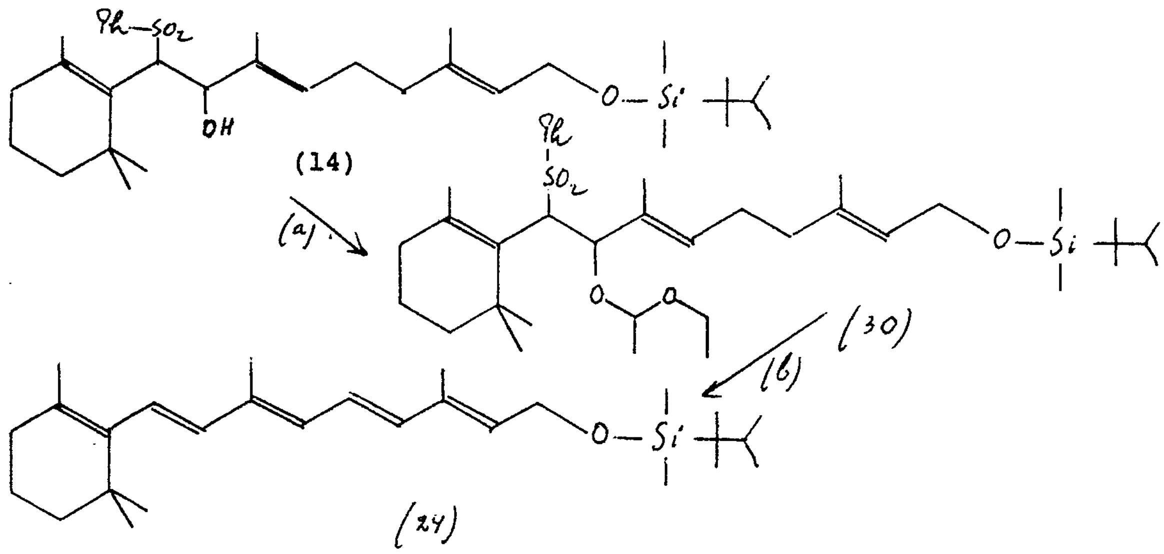


(a) Compound (14), prepared as described in Example I, in a quantity of 7.50g (12.75mmol) is dissolved into 50ml of methylenechloride. To this solution are added 2.44ml (22.5mmol) of ethyl vinyl ether and 32mg (0.13mmol) of pyridinium p-toluenesulphonate. The reaction mixture is stirred for 24hr, after which in addition 1.22ml (11.25mmol) of ethyl vinyl ether and 30mg (0.12mmol) of pyridinium p-toluenesulphonate are added. After stirring for another 24hrs, the reaction mixture is washed successively with 5% aqueous $NaHCO_3$-solution and with water. After drying and evaporation, the derived compound (30) is obtained in a yield of 8.12g. Identification by NMR. $^1$H-NMR($CDCl_3$): 0.01(s.6H); 0.85(s.6H); 0.89(d.6H); 0.6-2.3(m.32H); 3.18-3.85(3m.2H); 4.0-4.35(2m+2d.3H); 4.4-5.15(2d+2m.2H); 5.2-5.8(2m.2H); 7.4-8.12-(mdm.5H).

(b) Compound (30), thus obtained, is converted into the vitamin A derivative (24) by dissolving 1.1381g (1.72mmol) in 5ml of hexane. To this solution is added a solution of 482mg (6.88mmol) of $KOCH_3$ in 5ml hexane. The reaction mixture is refluxed (69°C) for 1.5hr and, after cooling down to room temperature, diluted with 20ml diethylether and washed with water. The ether layer is successively washed with 5% aqueous $NaHCO_3$-solution and with water (2x). After drying and evaporation the desired compound (24) is obtained in a yield of 0.7073g (79%); all-trans content 93%. The NMR spectrum corresponds with that of the vitamin A ether (24), obtained according to Example V.

## Claims

**1.** A sulfonyl compound having the general formula

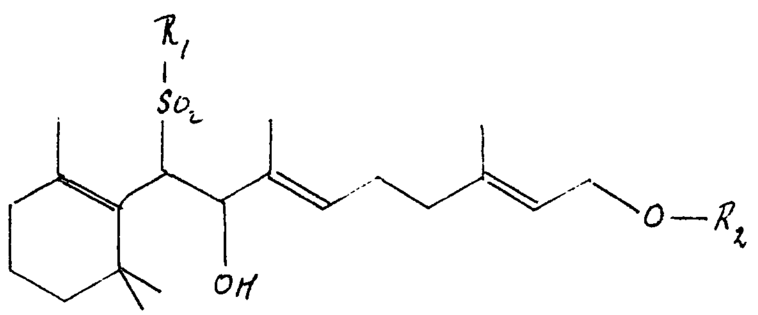

(I)

wherein

$R_1$ is an unsubstituted or substituted aryl group; and

$R_2$ is a $C_1$-$C_{20}$ alkyl group, a $C_2$-$C_{20}$ alkenyl group or a $C_2$-$C_{20}$ alkynyl group, which groups may be substituted with one or more substituents selected from the group consisting of $C_1$-$C_4$ alkoxy, halogen, unsubstituted aryl and substituted aryl; or wherein $R_2$ is a tri(hydrocarbyl)-

silyl group, an optionally lower-alkyl- substituted dihydro- or tetrahydropyran-2-yl group, an optionally lower-alkyl-substitute dihydro- or tetrahydro-fur-2-yl group, or a lower alkoxyalkylgroup.

**2.** A sulfonyl compound as claimed in claim 1, having the general formula

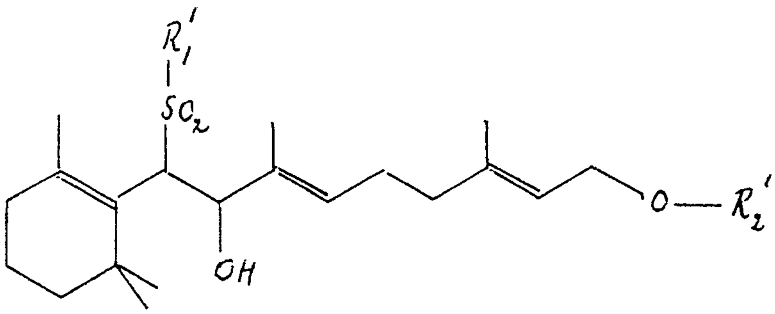
(II)

wherein

- $R_1$' is an unsubstituted phenyl group or a phenyl group substituted with 1-3 substituents selected from the group consisting of lower alkyl, lower alkoxy and halogen; and
- $R_2$' is a tri(hydrocarbyl)silyl group, wherein hydrocarbyl is selected from the group consisting of $C_1$-$C_8$ alkyl and phenyl; or wherein
- $R_2$' is a methyl group, a tert.($C_4$-$C_{12}$)alkyl group, a tetrahydropyran-2-yl group, a tetrahydrofur-2-yl group, or an ethoxyethyl group.

**3.** A method of preparing a sulfonyl compound as claimed in claim 1, characterized in that a compound of the general formula

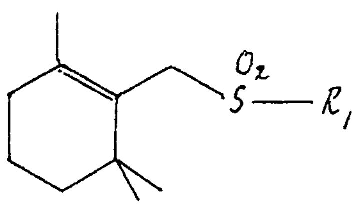
(III)

wherein $R_1$ has the meaning given in claim 1, is reacted with an aldehyde of the general formula

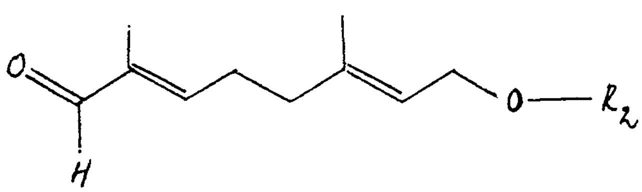
(IV)

wherein $R_2$ has the meaning given in claim 1,
with the aid of a base.

**4.** Aldehyde compound, suitable for the method as claimed in claim 3, having the general formula

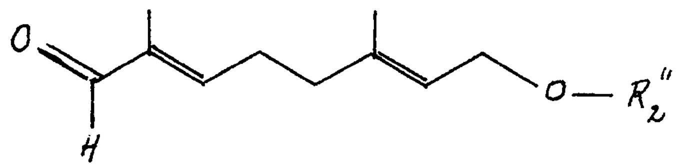

(XI)

wherein $R_2$"is a $C_1$-$C_{20}$ alkyl group, $C_2$-$C_{20}$ alkenyl group or a $C_2$-$C_{20}$ alkynyl groups, which groups may be substituted with one or more substituents selected from the group consisting of $C_1$-$C_4$ alkoxy, halogen, unsubstituted aryl and substituted aryl, preferably a methyl group or a tert.($C_4$-$C_{12}$)alkyl group; or

wherein $R_2$" is a tri(hydrocarbyl)silyl group, wherein hydrocarbyl is selected from the group consisting of $C_1$-$C_8$ alkyl and phenyl.

**5.** Use of a sulfonyl compound having the general formula I, presented in claim 1, for the preparation of vitamin A or an ester thereof, by successively

(i) protecting the free hydroxy group by converting said compound of formula I into a compound of the general formula

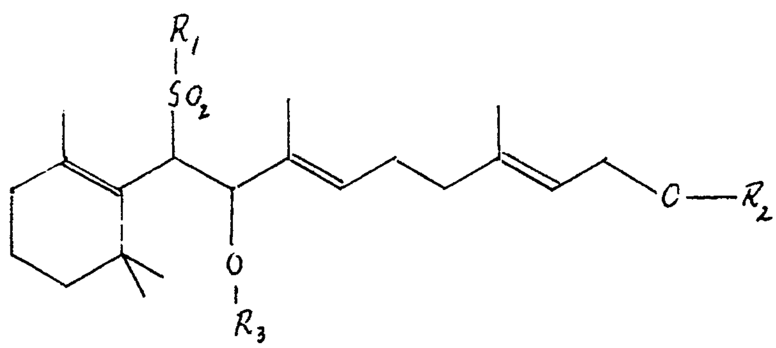

(V)

wherein

| | |
|---|---|
| $R_1$ and $R_2$ | have the meanings given in claim 1, and |
| $R_3$ | is an optionally lower-alkyl-substituted dihydro- or tetrahydro-pyran-2-yl group, an optionally lower-alkyl-substituted dihydro-or tetrahydro-fur-2-yl group, or a lower alkoxyalkyl group; |

(ii) removing $R_2$ from the above compound by converting the group $OR_2$ in said compound of formula V into a group $OR_4$,

wherein $R_4$ is a hydrogen atom or a lower alkanoyl group; and finally

(iii) converting the compound thus obtained by a double elimination reaction known per se into vitamin A or an ester thereof having the general formula

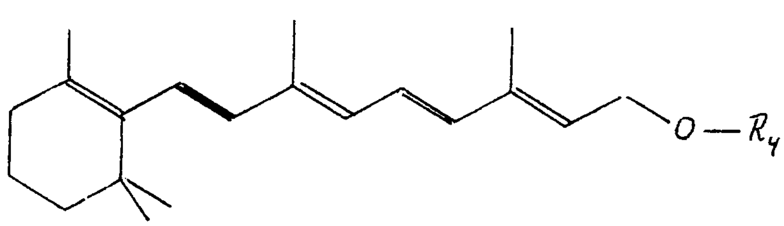

(VI)

**6.** A compound of the general formula V, presented in claim 5, wherein $R_1$ and $R_2$ have the meanings given in claim 1 and $R_3$ has the meaning given in claim 5, obtainable by employing the method as

described in claim 5, under (i).

7. Use of a sulfonyl compound having the general formula I, as presented in claim 1, for the preparation of vitamin A or an ester thereof, by successively
(i) protecting the free hydroxy group by converting the said compound of formula I into a compound of the general formula V, as presented in claim 5,
wherein $R_1$ and $R_2$ have the meanings given in claim 1 and $R_3$ has the meaning given in claim 5;
(ii) converting said compound of formula V by a double elimination reaction with the aid of a base into a vitamin A derivative of the general formula

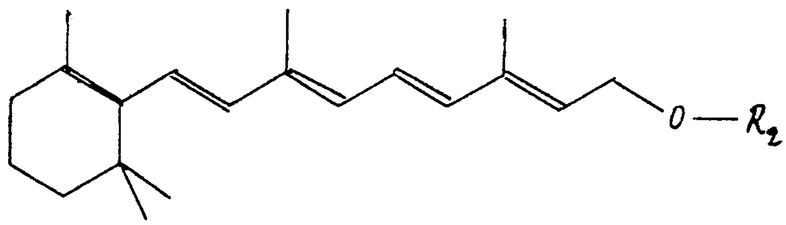

(VII)

and finally
(iii) removing $R_2$ from the above compound by converting the group $OR_2$ in said compound of formula VII into a group $OR_4$,
wherein $R_4$ has the meaning given in claim 5.

8. Use of a sulfonyl compound having the general formula I, as presented in claim 1, for the preparation of vitamin A or an ester thereof, by successively
(i) reacting said compound of formula I with a halogenating agent to form a compound of the general formula

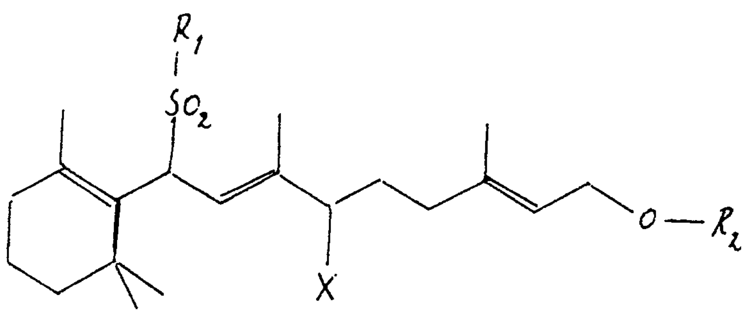

(VIII)

wherein
$R_1$ and $R_2$ have the meanings given in claim 1, and
X represents a halogen atom;
(ii) removing $R_2$ from the above compound by converting the group $OR_2$ in said compound of formula VIII into a group $OR_4$,
wherein $R_4$ has the meaning given in claim 5;
and finally
(iii) converting the compound thus obtained by a double elimination reaction known per se into vitamin A or an ester thereof having the general formula VI, as presented in claim 5.

9. A compound of the general formula VIII, presented in claim 8, wherein $R_1$ and $R_2$ have the meanings given in claim 1 and X represents a halogen atom, obtainable by employing the method as described in claim 8 under (i).

**10.** Use of a sulfonyl compound having the general formula I, presented in claim 1, for the preparation of vitamin A or an ester thereof, by successively

(i) reacting said compound of formula I with a halogenating agent to form a compound of the general formula VIII, as presented in claim 8, wherein $R_1$ and $R_2$ have the meanings given in claim 1, and X represents a halogen atom;

(ii) converting said compound of formula VIII by a double elimination reaction with the aid of a base into a vitamin A derivative of the general formula VII, as presented in claim 7;

and finally

(iii) removing $R_2$ from the above compound by converting the group $OR_2$ in said compound of formula VII into a group $OR_4$,

wherein $R_4$ has the meaning given in claim 5.

**11.** Use of a sulfonyl compound having the general formula I, presented in claim 1, for the preparation of vitamin A or an ether thereof, by successively

(i) reacting said compound of formula I with a halogenating agent to form a compound of the general formula VIII, as presented in claim 8, wherein $R_1$ and $R_2$ have the meanings given in claim 1, and X represents a halogen atom;

(ii) converting said compound of formula VIII by an elimination reaction with the aid of a base into a compound of the general formula

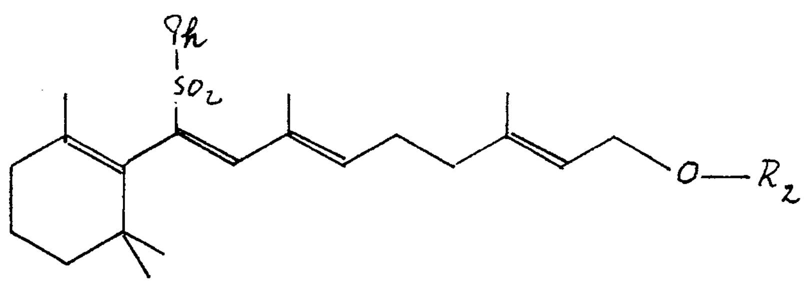

(X)

(iii) removing $R_2$ from the compound of formula X by converting the group $OR_2$ in said compound into a group $OR_4$, wherein $R_4$ has the meaning given in claim 5; and finally

(iv) converting the compound, thus obtained by a further elimination reaction with the aid of a base into vitamin A or an ester thereof, having the general formula VI, as presented in claim 5.

**12.** A method of preparing vitamin A or an ester thereof, characterized,

(a) in that a compound of the general formula III, as presented in claim 3, wherein $R_1$ has the meaning given in claim 1,

is reacted with an aldehyde of the general formula IV, as presented in claim 3, wherein $R_2$ has the meaning given in claim 1,

with the aid of a base;

(b) in that thereupon the sulfonyl compound thus obtained, having the general formula I, as presented in claim 1, is converted either into a compound of the general formula V, as presented in claim 5, or into a compound of the general formula VIII, as presented in claim 8, in which formulas $R_1$ and $R_2$ have the meanings given in claim 1, $R_3$ has the meaning given in claim 5 and X has the meaning given in claim 8;

after which the group $OR_2$ in said compound of general formula V or VIII, respectively, is converted into a group $OR_4$, wherein $R_4$ has the meaning given in claim 5; and

(c) in that finally the compound thus obtained is converted by a double elimination reaction known per se into vitamin A or an ester thereof having the general formula VI, as presented in claim 5.

**13.** A method of preparing vitamin A or an ester thereof, characterized,

(a) in that a compound of the general formula III, as presented in claim 3, wherein $R_1$ has the meaning given in claim 1,

is reacted with an aldehyde of the general formula IV, as presented in claim 3, wherein $R_2$ has the meaning given in claim 1,

with the aid of a base;

(b) in that thereupon the sulfonyl compound thus obtained, having the general formula I, as presented in claim 1, in converted either into a compound of the general formula V, as presented in claim 5, or into a compound of the general formula VIII, as presented in claim 8, in which formulas $R_1$ and $R_2$ have the meanings given in claim 1, $R_3$ has the meaning given in claim 5 and X has the meaning given in claim 8; and

(c) in that finally said compound of formula V or VIII, respectively, is converted by a double elimination reaction with the aid of a base into a vitamin A derivative of the general formula VII, as presented in claim 7;

after which the group $OR_2$ in said compound of formula VII in converted into a group $OR_4$, wherein $R_4$ has the meaning given in claim 5.

European Patent Office

# EUROPEAN SEARCH REPORT

Application Number

**EP 91 20 0666**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 348 813 (KURARAY CO.,LTD.)<br>* the whole document * | 1-11 | C 07 C 403/22<br>C 07 C 317/22<br>C 07 F 7/18<br>C 07 C 47/277 |
| A,D | JOURNAL OF ORGANIC CHEMISTRY. vol. 51, no. 20, 1986, EASTON US pages 3834 - 3838; JUNZO OTERA ET AL.: "STEREOCONTROLLED SYNTHESIS OF VITAMIN A THROUGH A DOUBLE ELIMINATION REACTION. A NOVEL CONVERGENT C10 + C10 ROUTE"<br>* the whole document * | 1,3 | |
| A,D | EP-A-0 187 259 (KURARAY CO.,LTD.)<br>* the whole document * | 1-11 | |
| A,D | JOURNAL OF THE AMERICAN CHEMICAL SOCIETY. vol. 106, no. 12, 13 June 1984, GASTON, PA US pages 3670 - 3672; TADAKATSU MANDAI ET AL.: "NOVEL SYNTHESIS OF ACETYLENES AND POLYENES VIA DESULFONYLATION REACTION"<br>* the whole document * | 1,10 | |
| A | CHEMISTRY LETTERS. 1985, TOKYO JP pages 1883 - 1886; JUNZO OTERA ET AL.: "THE HIGHLY STEREOSELECTIVE SYNTHESIS OF ALL-TRANS AND 13-CIS VITAMIN A VIA DOUBLE ELIMINATION REACTION"<br>* the whole document * | 1 | TECHNICAL FIELDS SEARCHED (Int. Cl.5)<br>C 07 C<br>C 07 F |
| X | GB-A-2 135 990 (EISAI CO LTD)<br>* the whole document * | 4 | |
| X | CHEMICAL ABSTRACTS, vol. 107, no. 19, 09 November 1987 Columbus, Ohio, USA INOUE SEIICHI ET AL.: "REGIO- AND STEREOSELECTIVE OXIDATION OF GEM-DIMETHYL OLEFINS VIA [2,3]-SIGMATROPIC REARRANGEMENT OF ALLYL AMINE OXIDES."<br>& CHEM. LETT. 1986, VOL.12 PAGES 2035-8 page 746; column 1; ref. no. 176241W EP 91200666030<br>* abstract * | 4 | |

The present search report has been drawn up for all claims

| Place of search | Date of completion of search | Examiner |
|---|---|---|
| Berlin | 13 May 91 | RUFET J.M.A. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document